**Europäisches Patentamt**

**European Patent Office**

**Office européen des brevets**

(19)

(11) Veröffentlichungsnummer : **0 421 122 B1**

(12) # EUROPÄISCHE PATENTSCHRIFT

(45) Veröffentlichungstag der Patentschrift :
**10.11.93 Patentblatt 93/45**

(51) Int. Cl.$^5$ : **C07C 25/02, C07C 17/12**

(21) Anmeldenummer : **90116630.6**

(22) Anmeldetag : **30.08.90**

(54) **Verfahren zur Herstellung von p-Chlortoluol.**

(30) Priorität : **15.09.89 DE 3930839**

(43) Veröffentlichungstag der Anmeldung :
**10.04.91 Patentblatt 91/15**

(45) Bekanntmachung des Hinweises auf die
Patenterteilung :
**10.11.93 Patentblatt 93/45**

(84) Benannte Vertragsstaaten :
**BE DE FR GB IT**

(56) Entgegenhaltungen :
**EP-A- 0 112 722**
**EP-A- 0 195 514**
**EP-A- 0 231 133**
**EP-A- 0 248 931**

(56) Entgegenhaltungen :
**GB-A- 2 155 009**
**SYNTHESIS Nr. 12, Dezember 1985, Seiten**
**1157,1158, Stuttgart, DE; K. SMITH: "Highly**
**para-Selective Mono-Chlorination of Aromatic**
**Compounds Under Mild Conditions by t-Butyl**
**Hypochlorite in the Presence of Zeolites"**

(73) Patentinhaber : **BAYER AG**
**D-51368 Leverkusen (DE)**

(72) Erfinder : **Botta, Artur, Dr.**
**Bodelschwinghstrasse 119**
**D-4150 Krefeld (DE)**
Erfinder : **Buysch, Hans-Josef, Dr.**
**Brandenburger Strasse 28**
**D-4150 Krefeld (DE)**
Erfinder : **Puppe, Lothar, Dr.**
**Am Weiher 10a**
**D-5093 Burscheid (DE)**

## Beschreibung

Die Erfindung betrifft ein Verfahren zur Herstellung von p-Chlortoluol durch Chlorierung von Toluol in Gegenwart von Zeolith L und Dichlormethan und/oder Chloroform.

Die herkömmliche Chlorierung von Toluol in Gegenwart von Eisen führt zu einem Gemisch von Chlortoluolen, in dem das o-Isomere mit 2:1 gegenüber dem p-Isomeren überwiegt. Durch Cokatalyse mit Schwefel oder Schwefelverbindungen läßt sich dieses Verhältnis auf Werte von etwa 1,1:1 bis etwa 0,75:1 verschieben (EP 292 824, US 4.031.147, US 4.444.983). Es ist weiterhin bekannt, Toluol ohne Mitverwendung von Lösungsmitteln in Gegenwart von Zeolith X, Y oder L zu einem Chlortoluolgemisch mit einem o-/p-Verhältnis von 1:2 umzusetzen (EP 231 662, EP 112 722). Durch Cokatalyse mit einigen Alkoholen und/oder einigen Carbonsäuren kann dieses Verhältnis nochmals geringfügig auf etwa 1:3 verbessert werden (EP 248 931, EP 154 236).

Ein Verhältnis von bis zu 91 % p-Chlortoluol neben 9 % o-Chlortoluol bei einer Ausbeute von bis zu 88 % der theoretischen Ausbeute wird nach einer Vorschrift in Synthesis 1985, 1157/58 erreicht, wenn man mit t-Butylhypochlorit in Gegenwart von Na-Faujasit in einem Reaktionsmedium von Dichlormethan und Diethylether arbeitet. Organische Hypochlorite sind zwar sehr selektiv, aber für eine technische Anwendung unangemessen teuer. Die Steigerung der para-Selektivität ist um so stärker ausgeprägt, je höher der Anteil an Ether im Gemisch ist. Ein anderes selektivitätssteigerndes Reaktionsmedium ist Acetonitril an Stelle von Diethylether.

Für die Mehrzahl der technischen Anwendungen ist das p-Chlortoluol das höherwertige Produkt; daher war es wünschenswert, die bisher bekannt gewordenen Herstellungsverfahren in bezug auf die p-Selektivität weiter zu verbessern.

Es wurde ein Verfahren zur Herstellung von p-Chlortoluol durch katalysierte Umsetzung von Toluol mit einem Chlorierungsmittel aus der Gruppe von Chlor, Sulfurylchlorid, N-Chloramine und N-Chloramide gefunden, das dadurch gekennzeichnet ist, daß als Katalysator ein Metallkationen enthaltender Zeolith L eingesetzt wird und in Gegenwart von Dichlormethan und/oder Chloroform gearbeitet wird.

Mit Hilfe des erfindungsgemäßen Verfahrens kann das o-/p-Isomerenverhältnis der Chlortoluole bis in Bereiche von 1:4 und sogar 1:5,5 verschoben werden. Dieser Befund ist außerordentlich überraschend, da nicht zu erwarten war, daß die Mitverwendung von Dichlormethan und/oder Chloroform, welche für gewöhnlich nur als Verdünnungsmittel betrachtet werden, überhaupt einen Einfluß auf das o-/p-Isomerenverhältnis haben könnte. Dieser Befund ist weiterhin überraschend, als vergleichbare Lösungsmittel, wie Tetrachlorkohlenstoff, 1,1,1-Trichlorethan oder 1,2-Dichlorpropan eine solche weitergehende Verschiebung des Isomerenverhältnisses nicht bewirken.

Die weitergehende Verschiebung des Isomerenverhältnisses im Rahmen des erfindungsgemäßen Verfahrens stellt bei einem Großprodukt wie p-Chlortoluol eine beträchtliche Verbesserung der Wirtschaftlichkeit dar.

Als Chlorierungsmittel im erfindungsgemäßen Verfahren kommen Chlor und Chlor abgebende Substanzen wie Sulfurylchlorid, N-Chloramine oder N-Chloramide in Frage. In bevorzugter Weise wird elementares Chlor eingesetzt. Das Halogenierungsmittel wird in der Regel im stöchiometrischem Verhältnis zum Toluol eingesetzt, d. h. im Molverhältnis von etwa 1:1. Von diesem stöchiometrischen Verhältnis kann bis zu 50, bevorzugt bis zu 30 % nach oben oder bis 60 %, bevorzugt bis 40 % nach unten abgewichen werden, um in einer dem Fachmann bekannten Weise den Umsetzungsgrad und/oder die p-Selektivität nach vorliegendem Bedürfnis zu beeinflussen.

Das erfindungsgemäße Verfahren wird in Gegenwart von Zeolith L als Katalysator durchgeführt, bei dem mindestens ein Teil aller austauschbaren Kationen Metallkationen sind.

Zeolithe sind kristalline Alumosilikate, die aus einem Netzwerk von $SiO_4$- und $AlO_4$-Tetraedern aufgebaut sind. Die einzelnen Tetraeder sind mit Sauerstoffbrücken über die Ecken untereinander verknüpft und bilden ein räumliches Netzwerk, das von Kanälen und Hohlräumen durchzogen ist. Als Ausgleich für die negative Ladung des Gerüsts sind austauschbare Kationen eingelagert. Si und Al in den Zeolithen können zumindest teilweise durch andere Elemente ersetzt sein. Eine ausführliche Darstellung von Zeolithen ist beispielsweise in der Monographie von D. W. Breck "Zeolite Molecular Sieves, Structure, Chemistry and Use", J. Wiley and Sons, New York, 1974 gegeben.

Die für das erfindungsgemäße Verfahren in Frage kommenden Zeolithe vom L-Typ weisen Porenweiten von ca. 7 Å und ein Si:Al-Verhältnis von 2,6-3,5:1 auf. Solche Zeolithe sind dem Fachmann grundsätzlich bekannt.

Für den erfindungsgemäßen Einsatz kommt ein Zeolith L in Frage, in dem 60-100 Äquivalent-%, bevorzugt 80-100 Äquivalent-%, besonders bevorzugt 90-100 Äquivalent-% aller Kationen Metallkationen sind. Als solche Metallkationen kommen beispielsweise in Frage: Kationen der Alkalimetalle Li, Na, K, Rb, Cs, das Ammoniumkation, Kationen der Erdalkalimetalle, wie Ca, Mg, Sr, Ba, der Selten-Erdmetalle, wie La, Ce und weiterer Metalle, wie Cu, Fe, Zn, Mn, Cr, Co, Ni, Ti, Ag und Pb. Selbstverständlich können auch Gemische solcher Kationen vorliegen. In bevorzugter Weise liegen die Kationen der Metalle K, Rb, Cs, Ca, Sr, Ba, Ag, Pb oder Gemische von ihnen vor. In besonders bevorzugter Weise liegen Kationen der Metalle K, Rb, Ca, Sr, Ba, Pb, Ag oder Gemische von ihnen vor.

Der Zeolith-Katalysator wird in einer Menge von 1-100 Gew.-%, bevorzugt 3-50 Gew.-%, besonders bevorzugt 5-30 Gew.-%, bezogen auf das Gewicht des Toluols, eingesetzt.

Die Form des verwendeten Zeolithkatalysators ist für das erfindungsgemäße Verfahren im allgemeinen nicht kritisch. In der Regel - insbesondere bei einer batch-Verfahrensweise - kann er in reiner Form als Pulver eingesetzt werden. Selbstverständlich ist es aber auch möglich - beispielsweise für eine kontinuierliche Verfahrensweise in der Gas-, Flüssig- oder Rieselphase, wo der Katalysator gegebenenfalls in einer stationären bzw. Festbettphase angeordnet wird, ihn in stückiger bzw. geformter Gestalt einzusetzen, um eine bessere Trennung vom Reaktionsgut zu gewährleisten. Dabei können übliche dem Fachmann geläufige Binde- bzw. Formungshilfsmitel mitverwendet werden, die gegenüber dem Halogenierungsagens inert sind, z.B. $SiO_2$, $Al_2O_3$, Tonerde, Graphit, in einer Menge von 0,1-80 %, bevorzugt 2-30 %, bezogen auf die Masse des reinen Zeoliths.

Das erfindungsgemäße Verfahren ist weiterhin durch die Mitverwendung von Dichlormethan und/oder Chloroform gekennzeichnet. Hierbei werden Dichlormethan und/oder Chloroform in der 0,05-100fachen, bevorzugt in der 0,2-50fachen, besonders bevorzugt in der 0,5-10fachen Gewichtsmenge, bezogen auf die des Toluols, eingesetzt. In bevorzugter weise wird Dichlormethan oder ein mindestens 50 Gew.-%, bevorzugt mindestens 70 Gew.-%, besonders bevorzugt mindestens 90 Gew.-%, Dichlormethan enthaltendes Gemisch als Verdünnungsmittel eingesetzt.

Die neben dem Dichlormethan und/oder Chloroform verwendbaren Verdünnungsmittel sind z.B. solche, die gegenüber den Chlorierungsmitteln inert sind, etwa Kohlenwasserstoffe oder Halogenkohlenwasserstoffe, wie Petrolether, Tetrachlorkohlenstoff, 1,1,1-Trichlorethan, 1,2-Dichlorethan, 1,2-Dichlorpropan, Perchlorethan, Perchlorethylen, niedere Carbonsäuren, wie Essigsäure und andere dem Fachmann bekannte Verdünnungsmittel. Da solche mitverwendbaren Verdünnungsmittel jedoch keinen zusätzlichen Effekt ergeben, ist zugunsten einer leichteren Aufarbeitung der alleinige Einsatz von Dichlormethan und/oder Chloroform, bevorzugt von Dichlormethan, bevorzugt.

Es ist weiterhin zulässig, dem Fachmann bekannte Co-Katalysatoren, wie niedere Alkohole, niedere Carbonsäuren, Schwefelverbindungen und/oder quartäre Ammoniumsalze im erfindungsgemäßen Verfahren zu verwenden. In bevorzugter Weise wird ohne solche Co-Katalysatoren gearbeitet.

Das erfindungsgemäße Verfahren wird bei einer Temperatur von -20°C bis +120°C, bevorzugt 0-90°C, besonders bevorzugt 10-70°C, durchgeführt. Der Druck ist für den Ablauf des erfindungsgemäßen Verfahrens nicht kritisch und hat beispielsweise nur Bedeutung, um bei erhöhter Temperatur das Dichlormethan größtenteils in der flüssigen Phase des Reaktionsgemisches zu halten. Hierbei wird in bevorzugter Weise unter dem sich einstellenden Eigendruck des Reaktionsgemisches gearbeitet. Ein verminderter Druck könnte dann Bedeutung haben, wenn bei einer Temperatur unterhalb des Siedepunktes des Verdünnungsmittels unter Rückflußbedingungen zur Kontrolle der Reaktionstemperatur (Siedekühlung) gearbeitet werden soll. Diese Zusammenhänge sind dem Fachmann geläufig. In allen Fällen, in denen die Arbeitsweise unter Normaldruck möglich ist, ist eine solche Arbeitsweise bevorzugt.

Bei der Durchführung des erfindungsgemäßen Verfahrens in diskontinuierlicher Arbeitsweise kann beispielsweise das Toluol mit dem Verdünnungsmittel gemischt werden (gegebenenfalls unter Zusatz eines der genannten Co-Katalysatoren), woraufhin der Zeolith-Katalysator in Pulverform oder in granulierter Form zugefügt wird. Unter Rühren wird sodann das Chlorierungsmittel in dem Maße, wie es verbraucht wird, bei Reaktionstemperatur in die flüssig-disperse Phase eingeleitet.

Für die kontinuierliche Durchführung eignen sich beispielsweise Säulenapparaturen, in denen der Zeolith-Katalysator in stückiger oder granulierter Form oder in Pulverform auf verschiedenen Böden angeordnet wird. Über eine solche Anordnung werden das Toluol-Dichlormethan-Gemisch und das Chlorierungsmittel entweder im Gleichstrom oder im Gegenstrom geführt.

Die Isolierung und Reinigung des p-Chlortoluols geschieht, häufig nach Abtrennung des Katalysators, in der Regel durch Destillation unter normalem oder vermindertem Druck.

Der entweder als Destillationsrückstand oder als Filtrationsrückstand hinterbleibende Zeolith-Katalysator kann im allgemeinen ohne weitere Aktivierung mehrfach erneut erfindungsgemäß eingesetzt werden. Für den Fall, daß nach mehrmaliger Wiederverwendung eine Aktivitätsminderung beobachtet wird, kann der Zeolith-Katalysator nach einem üblichen Verfahren, beispielsweise durch Calcinierung bei erhöhter Temperatur (etwa 400-600°C) reaktiviert werden.

Beispiele

Alle in den folgenden Beispielen genannten Zeolithe wurden vor ihrem Einsatz 2-3 Stunden bei 400°C in einem Muffelofen aktiviert.

Beispiele 1-7

In einer Dreihalsrührapparatur aus Glas (Rundkolben) leitete man in eine Suspension von 9,2 g (20 %, bezogen auf Toluol) K-Zeolith L in Pulverform, 46 g Toluol (0,5 Mol) und X g $CH_2Cl_2$ bei 40°C unter Rühren innerhalb von 5 Stunden 35,5 g (0,5 Mol) Chlor ein. Anschließend rührte man noch 15 Minuten unter Durchleiten von Stickstoff nach. Die Ergebnisse der gaschromatographischen Analyse gehen aus der Tabelle 1 hervor (Angaben in allen Tabellen in Flächen-%).

Beispiele 8-11

Bei gleicher Verfahrensweise wie in Beispiel 6 wurde die Menge an Katalysator K-L von 5-25 Gew.-% variiert. Die Ergebnisse der gaschromatographischen Untersuchung sind aus Tabelle 2 ersichtlich.

Beispiele 12-15 (Vergleichsbeispiele)

Bei gleicher Verfahrensweise wie in den Beispielen 1-7 wurde bei Einsatz von 50 ml Dichlormethan und einer Reaktionstemperatur von 40°C die Art des Katalysators variiert. Nähere Einzelheiten sowie die Ergebnisse der gaschromatographischen Analyse zur Zusammensetzung des Reaktionsgemisches sind in Tabelle 3 zusammengestellt.

EP 0 421 122 B1

<u>Tabelle 1</u>

| Bsp. | X g $CH_2Cl_2$ | Toluol | Chlortoluol 2- | Chlortoluol 4- | Benzyl-chlorid | Dichlortoluol 2,4- | Dichlortoluol 3,4- | Rest | 4-Selekt. |
|------|------|--------|-----|-----|--------|------|------|------|-----------|
| 1 | 2,3 | 10,5 | 28,5 | 58,6 | 0,6 | 0,6 | 0,2 | 0,9 | 65 |
| 2 | 4,6 | 5,9 | 31,6 | 59,1 | 1,7 | 0,7 | 0,2 | 0,9 | 63 |
| 3 | 9,2 | 6,5 | 27,3 | 64,1 | 0,6 | 0,6 | 0,2 | 0,7 | 69 |
| 4 | 23 | 6,1 | 22,2 | 69,0 | 0,6 | 0,8 | 0,2 | 1,3 | 73 |
| 5 | 46 | 11,5 | 17,4 | 70,3 | 0,2 | 0,3 | – | 0,4 | 79 |
| 6 | 69 | 10,8 | 15,6 | 72,6 | 0,2 | 0,3 | 0,2 | 0,3 | 81,4 |
| 7 | 138 | 8,7 | 14,9 | 75,6 | 0,4 | 0,3 | – | 0,1 | 83 |

Tabelle 2

| Bsp. | Gew.-% Kat. | Toluol | Chlortoluol 2- | Chlortoluol 4- | Benzyl-chlorid | Dichlortoluol 2,4- | Dichlortoluol 3,4- | Rest | 4-Selekt. |
|------|------|------|------|------|------|------|------|------|------|
| 8 | 5 | 12,7 | 16,9 | 68,3 | 0,9 | 0,4 | 0,09 | 0,7 | 78 |
| 9 | 10 | 7,8 | 17,2 | 73,7 | 0,5 | 0,4 | 0,09 | 0,3 | 80 |
| 10 | 15 | 5,4 | 17,3 | 75,6 | 0,7 | 0,5 | 0,08 | 0,4 | 80 |
| 6 | 20 | 10,8 | 15,6 | 72,6 | 0,2 | 0,3 | 0,2 | 0,3 | 81,4 |
| 11 | 25 | 9,1 | 16,8 | 73,4 | 0,2 | 0,3 | - | 0,2 | 81 |

EP 0 421 122 B1

Tabelle 3

| Bsp. | Kat. | Toluol | Chlortoluol 2- | Chlortoluol 4- | Benzyl-chlorid | Dichlortoluol 2,4- | Dichlortoluol 3,4- | Rest | 4-Selekt. |
|---|---|---|---|---|---|---|---|---|---|
| 12 | ohne | 27,60 | 0,67 | 0,41 | 62,37 | 5,54 | - | 3,41 | 0,6 % |
| 13 | 1 % FeCl3 | 21,86 | 46,76 | 18,26 | - | 6,74 | 2,05 | 4,33 | 23,4 % |
| 14 | H-Mordenit | 14,76 | 54,18 | 26,39 | - | 1,62 | 0,34 | 2,71 | 31,0 % |
| 15 | H-ZSM-5 | 29,1 | 11,51 | 6,02 | 41,75 | 0,16 | 0,03 | 11,43 | 8,5 % |
| 16 | Rb-L | 15,54 | 15,05 | 67,64 | 0,58 | 0,37 | 0,08 | 0,74 | 80,1 % |
| 17 | Ag-L | 27,44 | 13,10 | 58,13 | 0,41 | 0,19 | 0,05 | 0,68 | 80,1 % |
| 18 | Pb-L | 7,35 | 18,47 | 72,32 | 0,46 | 0,42 | 0,08 | 0,90 | 78,1 % |

Beispiele 16-18

Es wurde analog den Beispielen 1-7 gearbeitet, wobei bei einer Reaktionstemperatur von 40°C und beim Einsatz von 50 ml Dichlormethan die Metallkationen des Zeoliths L variiert wurden. Weitere Einzelheiten sowie die Ergebnisse der gaschromatographischen Untersuchungen sind ebenfalls aus Tabelle 3 ersichtlich.

Beispiele 19-24

Die Chlorierungsreaktion an Toluol wurde analog den Beispielen 1-7 mit K-L als Katalysator, einer Reaktionstemperatur von 20°C und 50 ml an verschiedenen Lösungsmitteln durchgeführt. Die Art des Lösungsmittels und nähere Einzelheiten sowie die gaschromatographischen Analysenbefunde über die Zusammensetzung der Reaktionsgemische sind in Tabelle 4 zusammengestellt.

Beispiele 25-30

Bei gleicher Verfahrensweise wie in Beispiel 6 wurde die Toluolchlorierung bei einer Reaktionstemperatur von 40°C in 69 g eines Gemisches aus Dichlormethan und Chloroform durchgeführt. Die Einzelheiten über das Mischungsverhältnis sowie die Ergebnisse der gaschromatographischen Untersuchung der Reaktionsgemische gehen aus der Tabelle 5 hervor.

Tabelle 4

| Bsp. | Lösungsmittel | Toluol | Chlortoluol 2- | Chlortoluol 4- | Benzyl-chlorid | Dichlortoluol 2,4- | Dichlortoluol 3,4- | Rest | 4-Selekt. |
|---|---|---|---|---|---|---|---|---|---|
| 19 | Cyclohexan | 12,66 | 32,02 | 53,07 | - | 0,53 | 0,12 | 1,60 | 60,8 |
| 20 | n-Hexan | 66,20 | 12,26 | 19,18 | 0,20 | 0,25 | 0,08 | 1,83 | 56,7 |
| 21 | 1,2-Dichlor-propan | 16,45 | 20,47 | 54,28 | 1,18 | 0,94 | - | 6,68 | 64,9 |
| 22 | 1,1,1-Tri-chlorethan | 44,29 | 14,70 | 19,91 | 9,53 | 0,21 | 0,11 | 11,25 | 35,7 |
| 23 | CHCl$_3$ | 40,80 | 14,70 | 43,57 | - | 0,42 | 0,06 | 0,45 | 73,6 |
| 24 | CCl$_4$ | 8,49 | 33,83 | 55,90 | - | 0,72 | 0,18 | 0,88 | 61,1 |

Beispiel 31

In einem Planschliffreaktor aus Glas (Höhe 25 cm, Durchmesser 9 cm) mit Rührer, Thermometer, Rückflußkühler und einem bis zum Boden reichenden Gaseinleitungsrohr gab man 368 g (4 Mol) Toluol und 1104 g Dichlormethan zusammen und fügte 73,6 g pulverförmigen K-Zeolith L zu. Dann wurden unter Rühren bei 40°C insgesamt 312 g (4,4 Mol) $Cl_2$-Gas innerhalb von 10 Stunden in die Suspension eingeleitet. Nach Einleiten von 90 % (255,6 g), 100 % (284 g) und 110 % (312 g) der theoretisch erforderlichen $Cl_2$-Menge wurden Proben zur Bestimmung des Umsatzes entnommen. Die Ergebnisse der gaschromatographischen Analyse der Produktzusammensetzung sind aus der Tabelle 6 ersichtlich.

Beispiel 32

Analog der Arbeitsweise von Beispiel 31 wurden in einer 250 ml Planschiffapparatur (Höhe 11 cm, Durchmesser 5,5 cm) 55,3 g (0,6 Mol) Toluol in 120 ml Dichlormethan in Gegenwart von 13.0 g K-Zeolith L-Granulat (gebunden mit 15 % $SiO_2$) bei 40°C in 6 h mit $Cl_2$ umgesetzt. Die Probenahme für die gaschromatographische Untersuchung erfolgte bei Einleitung von 90 % (38,3 g), 100 % (42,6 g) sowie 110 % (46,9 g) $Cl_2$.

Beispiel 33

Die Durchführung erfolgte analog Beispiel 31 unter Verwendung von 15,8 g K/Na-Zeolith L (Granulat, gebunden mit 30 % $Al_2O_3$).

Die Ergebnisse der beiden Beispiele 32 und 33 sind in Tabelle 7 zusammengefaßt.

EP 0 421 122 B1

Tabelle 5

| Bsp. | Gew.-% CHCl$_3$ in CH$_2$Cl$_2$ | Toluol | Chlortoluol 2- | 4- | Benzyl-chlorid | Dichlortoluol 2,4- | 3,4- | Rest | 4-Selekt. |
|---|---|---|---|---|---|---|---|---|---|
| 25 | 100 | 9,24 | 23,39 | 64,74 | 0,47 | 0,56 | 0,13 | 1,47 | 71,3 |
| 26 | 50 | 5,33 | 19,57 | 70,51 | 1,95 | 0,46 | 0,07 | 2,11 | 74,5 |
| 27 | 25 | 9,34 | 17,32 | 70,28 | 0,68 | 0,47 | 0,15 | 1,76 | 77,5 |
| 28 | 10 | 14,06 | 15,91 | 66,05 | 1,09 | 0,36 | 0,12 | 2,41 | 76,9 |
| 29 | 5 | 16,47 | 15,72 | 66,57 | 0,30 | 0,33 | 0,06 | 0,55 | 79,7 |
| 30 | 2 | 9,56 | 16,74 | 72,04 | 0,15 | 0,30 | – | 1,21 | 79,7 |
| 6 | 0 | 10,8 | 15,6 | 72,6 | 0,20 | 0,3 | 0,2 | 0,30 | 81,4 |

Tabelle 6

| Bsp. | Probe bei | Toluol | Chlortoluol 2- | 4- | Benzyl-chlorid | Dichlortoluol 2,4- | 3,4- | Rest | 4-Selekt. |
|---|---|---|---|---|---|---|---|---|---|
| 31 | 90 % Cl$_2$ | 21,61 | 12,41 | 65,41 | 0,24 | 0,13 | – | 0,20 | 83,4 |
| | 100 % Cl$_2$ | 11,83 | 13,99 | 73,53 | 0,30 | 0,20 | – | 0,15 | 83,4 |
| | 110 % Cl$_2$ | 1,94 | 15,42 | 81,61 | 0,35 | 0,43 | 0,05 | 0,20 | 83,2 |

Tabelle 7

| Bsp. | Probe bei % Cl$_2$ | Toluol | Chlortoluol 2- | Chlortoluol 4- | Benzyl-chlorid | Dichlortoluol 2,4- | Dichlortoluol 3,4- | Rest | 4-Selekt. |
|---|---|---|---|---|---|---|---|---|---|
| 32 | 90 | 14,0 | 16,4 | 67,5 | 0,6 | 0,2 | 0,1 | 1,2 | 79,8 |
|  | 100 | 4,5 | 18,2 | 74,6 | 0,7 | 0,2 | 0,3 | 1,5 | 79,9 |
|  | 110 | - | 17,0 | 76,0 | 0,6 | 1,2 | 1,9 | 3,3 | 81,4 |
| 33 | 90 | 19,0 | 20,6 | 58,7 | 0,4 | 0,1 | 0,2 | 1,0 | 73,4 |
|  | 100 | 10,4 | 23,0 | 64,3 | 0,4 | 0,2 | 0,3 | 1,4 | 73,0 |
|  | 110 | 0,9 | 25,3 | 70,0 | 0,4 | 0,6 | 0,8 | 2,0 | 70,1 |

EP 0 421 122 B1

**Patentansprüche**

1. Verfahren zur Herstellung von p-Chlortoluol durch katalysierte Umsetzung von Toluol mit einem Chlorierungsmittel aus der Gruppe von Chlor, Sulfurylchlorid, N-Chloramine und N-Chloramide, dadurch gekennzeichnet, daß als Katalysator ein Metallkationen enthaltender Zeolith L eingesetzt wird und in Gegenwart von Dichlormethan und/oder Chloroform gearbeitet wird.

2. Verfahren nach Anspruch 1, dadurch gekennzeichnet, daß der Katalysator in einer Menge von 1-100 Gew.-%, bevorzugt 3-50 Gew.-%, besonders bevorzugt 5-30 Gew.-%, bezogen auf das Gewicht des Toluols eingesetzt wird.

3. Verfahren nach Anspruch 1, dadurch gekennzeichnet, daß im Zeolith L 60-100 Äquivalent-%, bevorzugt 80-100 Äquivalent-%, besonders bevorzugt 90-100 Äquivalent-%, aller Kationen Metallkationen sind.

4. Verfahren nach Anspruch 1, dadurch gekennzeichnet, daß als Metallkationen die der Alkalimetalle, der Erdalkalimetalle, der Seltenerdmetalle, des Kupfers, Eisens, Zinks, Mangans, Chroms, Kobalts, Nickels, Titans, Silbers oder Bleis oder eines Gemisches von ihnen, bevorzugt die Kationen von K, Rb, Cs, Ca, Sr, Ba, Ag, Pb oder Gemische von ihnen, besonders bevorzugt Kationen von K, Rb, Ca, Sr, Ba, Pb, Ag oder Gemische von ihnen eingesetzt werden.

5. Verfahren nach Anspruch 1, dadurch gekennzeichnet, daß Dichlormethan und/oder Chloroform in der 0,05-100fachen, bevorzugt in der 0,2-50fachen, besonders bevorzugt in der 0,5-10fachen, Gewichtsmenge, bezogen auf die des Toluols, eingesetzt wird.

6. Verfahren nach Anspruch 1, dadurch gekennzeichnet, daß bei einer Temperatur im Bereich von -20°C bis +120°C, bevorzugt 0-90°C, besonders bevorzugt 10-70°C, gearbeitet wird.

7. Verfahren nach Anspruch 1, dadurch gekennzeichnet, daß in Gegenwart von Dichlormethan oder einem mindestens 50 Gew.-%, bevorzugt mindestens 70 Gew.-%, besonders bevorzugt mindestens 90 Gew.-%, Dichlormethan enthaltendem Gemisch als Verdünnungsmittel gearbeitet wird.

8. Verfahren nach Anspruch 1, dadurch gekennzeichnet, daß elementares Chlor als Chlorierungsmittel eingesetzt wird.

**Claims**

1. Process for the preparation of p-chlorotoluene by catalysed reaction of toluene with a chlorinating agent from the comprising chlorine, sulphuryl chloride, N-chloroamines and N-chloroamides, characterized in that a zeolite L containing metal cations is employed as the catalyst and the reaction is carried out in the presence of methylene chloride and/or chloroform.

2. Process according to Claim 1, characterized in that the catalyst is employed in an amount of 1-100% by weight, preferably 3-50% by weight, particularly preferably 5-30% by weight, based on the weight of the toluene.

3. Process according to Claim 1, characterized in that 60-100 equivalent %, preferably 80-100 equivalent % and particularly preferably 90-100 equivalent % of all the cations in zeolite L are metal cations.

4. Process according to Claim 1, characterized in that the metal cations employed are those of the alkali metals, the alkaline earth metals, rare earth metals, copper, iron, zinc, manganese, chromium, cobalt, nickel, titanium, silver or lead or a mixture of these, preferably the cations of K, Rb, Cs, Ca, Sr, Ba, Ag or Pb or mixtures of these, particularly preferably cations of K, Rb, Ca, Sr, Ba, Pb or Ag or mixtures of these.

5. Process according to Claim 1, characterized in that methylene chloride and/or chloroform is employed in 0.05-100 times, preferably in 0.2-50 times and particularly preferably in 0.5-10 times the amount by weight of toluene.

6. Process according to Claim 1, characterized in that the reaction is carried out at a temperature in the range from -20°C to +120°C, preferably 0-90°C and particularly preferably 10-70°C.

7. Process according to Claim 1, characterized in that the reaction is carried out in the presence of methylene chloride or a mixture containing at least 50% by weight, preferably at least 70% by weight and particularly preferably at least 90% by weight of methylene chloride, as the diluent.

8. Process according to Claim 1, characterized in that elemental chlorine is employed as the chlorinating agent.


**Revendications**

1. Procédé de production de p-chlorotoluène par réaction catalysée de toluène avec un agent de chloration du groupe du chlore, du chlorure de sulfuryle, de N-chloramines et de N-chloramides, caractérisé en ce qu'on utilise comme catalyseur une zéolite L contenant des cations métalliques et on opère en présence de dichlorométhane et/ou de chloroforme.

2. Procédé suivant la revendication 1, caractérisé en ce qu'on utilise le catalyseur en une quantité de 1 à 100 % en poids, de préférence de 3 à 50 % en poids, notamment de 5 à 30 % en poids, par rapport au poids du toluène.

3. Procédé suivant la revendication 1, caractérisé en ce que dans la zéolite L, 60 à 100 équivalents %, de préférence 80 à 100 équivalents %, notamment 90 à 100 équivalents %, de tous les cations sont des cations métalliques.

4. Procédé suivant la revendication 1, caractérisé en ce qu'on utilise comme cations métalliques les cations des métaux alcalins, des métaux alcalino-terreux, des métaux des terres rares, de cuivre, de fer de zinc, de manganèse, de chrome, de cobalt, de nickel, de titane, d'argent ou de plomb ou d'un mélange de ces métaux, de préférence les cations de K, Rb, Cs, Ca, Sr, Ba, Ag, Pb ou des mélanges de ces cations, notamment des cations de K, Rb, Ca, Sr, Ba, Pb, Ag ou des mélanges de ces cations.

5. Procédé suivant la revendication 1, caractérisé en ce qu'on utilise le dichlorométhane et/ou le chloroforme en quantité en poids représentant 0,05 à 100 fois, de préférence 0,2 à 50 fois, notamment 0,5 à 10 fois, la quantité en poids de toluène.

6. Procédé suivant la revendication 1, caractérisé en ce qu'on opère à une température comprise dans la plage de -20 à +120°C, de préférence de 0 à 90°C, notamment de 10 à 70°C.

7. Procédé suivant la revendication 1, caractérisé en ce qu'on opère en présence de dichlorométhane ou d'un mélange contenant au moins 50 % en poids, de préférence au moins 70 % en poids, notamment au moins 90 % en poids, de dichlorométhane, en tant que diluant.

8. Procédé suivant la revendication 1, caractérisé en ce qu'on utilise du chlore élémentaire comme agent de chloration.